(19) Europäisches Patentamt
European Patent Office
Office européen des brevets

(11) Publication number: 0 121 254
B1

(12) **EUROPEAN PATENT SPECIFICATION**

(45) Date of publication of patent specification: **17.08.88**

(51) Int. Cl.$^4$: **C 12 Q 1/26**, C 12 Q 1/28, C 12 Q 1/44, C 12 Q 1/48, C 12 Q 1/60, C 12 Q 1/34, C 12 Q 1/52, C 12 Q 1/40

(21) Application number: **84103552.0**

(22) Date of filing: **30.03.84**

# (54) Process for determining substrate or enzymatic activity.

(30) Priority: **02.04.83 JP 58372/83**

(43) Date of publication of application: **10.10.84 Bulletin 84/41**

(45) Publication of the grant of the patent: **17.08.88 Bulletin 88/33**

(84) Designated Contracting States: **AT BE CH DE FR GB IT LI LU NL SE**

(56) References cited:
EP-A-0 044 615
EP-A-0 104 047
GB-A-2 028 500
US-A-4 172 765
US-A-4 241 178

PATENTS ABSTRACTS OF JAPAN, vol. 7, no. 7 (C-144)1152r, 12th January 1983

PATENTS ABSTRACTS OF JAPAN, vol. 8, no. 7 (C-204)1444r, 12th January 1984

**The file contains technical information submitted after the application was filed and not included in this specification**

(73) Proprietor: **WAKO PURE CHEMICAL INDUSTRIES, LTD.**
**10, Doshomachi-3-chome**
**Higashi-ku Osaka (JP)**

(72) Inventor: **Miyashita, Yoshinobu**
**14-17, Takadono-7-chome**
**Asahi-ku Osaka (JP)**
Inventor: **Hamanaka, Tadashi**
**52-6-506, Tsuchiyamacho**
**Nada-ku Kobe (JP)**
Inventor: **Kodera, Hiroyuki**
**83-1, Higashisonodacho-6-chome**
**Amagasaki-shi (JP)**
Inventor: **Matsuda, Ryosuke**
**7-C28-701, Shinsenrihigashimachi-2-chome**
**Toyonaka-shi (JP)**

(74) Representative: **Kinzebach, Werner, Dr. et al**
**Patentanwälte Reitstötter, Kinzebach und Partner Sternwartstrasse 4 Postfach 86 06 49**
**D-8000 München 86 (DE)**

EP 0 121 254 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for quantitatively determining a substrate or enzymatic activity in a body fluid. More particularly, this invention relates to a process for quantitatively determining a substrate or enzymatic activity after removing influences of a substance which is present in a body fluid and gives an error to the determination.

Recently, in the field of clinical examinations, there have widely been employed processes for measuring hydrogen perioxide produced by treating a substrate or a substance produced by an enzymatic reaction of a substrate with an oxidase as a process for measuring a component in a body fluid.

As a process for measuring hydrogen peroxide, there has mainly been employed a process comprising adding to a sample 4-aminoantipyrine and a phenol derivative, a naphthol derivative or an aniline derivative in the presence of peroxidase to produce a colored substance by oxidative condensation, followed by colorimetric determination. But with an increase of a demand for more precise determination, there has become a problem the presence of a substance in a body fluid, said substance giving an error to the determination. For example, there were found the following substances for giving errors to the determination, that is, free glycerol in a body fluid in the determination of triglyceride, cholesterol in the determination of cholesterol ester, sarcosine in the determination of creatine or creatinine, pyruvic acid in the enzymatic activity determination of glutamate pyruvate transaminase (hereinafter referred to as "GPT") or glutamate oxaloacetate transaminase (hereinafter referred to as "GOT"), and glucose in the enzymatic activity determination of amylase.

Various methods for decomposing or removing such substances as giving errors to the determination have been studied. For example, European Patent Publication No. 0054358 discloses a process for decomposing hydrogen peroxide by reacting hydrogen peroxide produced by oxidation of a free compound which gives an error to the determination with a phenol or aniline derivative in the presence of peroxidase to form non-coloring compound. Said European Patent Publication further provides a process for quantitative determination of a non-oxidizable analyte in a sample containing both said analyte and an enzymatically oxidizable derivative thereof, which comprises enzymatically oxidizing said derivative with an oxidase, decomposing hydrogen peroxide produced in the sample by said oxidase by a reaction with a phenol or aniline derivative in the presence of a peroxidase, converting the non-oxidizable analyte into a further quantity of said oxidizable derivative, and quantitatively determining said further quantity of said oxidizable derivative. In Chemical Abstracts 98:30600 m (1983), there is disclosed a process for determining a substrate or its enzymatic activity by the determination of $H_2O_2$ formed by reacting an oxidase with a product formed by the enzymatic reaction, characterized in that a sample is added to a first reagent containing an oxidase, peroxidase and a phenol, aniline or naphthol derivative, and the resulting mixture is mixed with a second reagent containing a coupler such as 4-aminoantipyrine, followed by colorimetric determination. According to these processes, a phenol or aniline derivative or a naphthol derivative is used for decomposing a substance which gives errors to the determination, but since the phenol, aniline or naphthol derivative is also used for producing a color with a coupler such as 4-aminoantipyrine, a partial consumption of the phenol, aniline or naphthol derivative for decomposing hydrogen peroxide makes it difficult to form uniform color producing conditions in the color developing reaction. On the other hand, if a large amount of the phenol, aniline or naphthol derivative is used for compensating the consumed amount for decomposing hydrogen peroxide which gives an error to the determination, the reagent blank value is undesirably raised, which results in making the determination with high accuracy impossible.

It is an object of this invention to provide a process for quantitatively determining a substrate or enzymatic activity in a body fluid with high accuracy by overcoming disadvantages of the prior art processes.

This invention provides a process for quantitatively determining a substrate or enzymatic activity in a body fluid which comprises adding to a sample to be measured a first reagent solution comprising 2-naphthol or a derivative thereof represented by the formula:

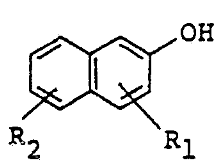

(I)

wherein $R_1$ and $R_2$ are independently hydrogen, a hydroxyl group, a carboxyl group or a sulfonic acid group, peroxidase, an oxidase, and if necessary one or more substances used for producing a substrate or used as substrates for oxidase or used for an enzymatic reaction of a substrate with the oxidase, so as to remove an influence of substance in the sample giving an error to the determination (an interfering substance), adding to the resulting mixture a second reagent solution comprising a coupler such as 4-aminoantipyrine, a developer selected from phenol derivatives, 1-naphthol derivatives and aniline derivatives, and an enzyme necessary for producing the same substance as the interfering substance (giving an error to the determination) in the case of determining the substrate, or one or more substances

used as substrates for the enzyme, the activity of which is to be determined, in the case of determining the enzymatic activity, followed by colorimetric determination.

As the substrate to be determined according to this invention, there are exemplified triglycedride, cholesterol ester, creatinine, creatine and the like.

As the enzyme, the activity of which is to be determined according to this invention, there are exemplified transaminases such as GPT, GOT; amylase, and the like.

According to this invention, the system containing the substance to be determined colorimetrically can be maintained under uniform conditions and there can be selected a suitable combination of color producing materials. Further, 2-naphthol or a derivative thereof represented by the formula (I) does not influence the color producing system comprising a coupler such as 4-aminoantipyrine and a phenol derivative, a 1-naphthol derivative or an aniline derivative. This is because the compound of the formula (I) has a higher oxidation-reduction potential to be oxidized by hydrogen peroxide in the presence of peroxidase than that of the color producing material (developer) such as a phenol derivative, a 1-naphthol derivative or an aniline derivative to be coupled with 4-aminoantipyrine, so that the phenol derivative, the 1-naphthol derivative or the aniline derivative seems to preferentially carry out the oxidative condensation with 4-aminoantipyrine.

As the compound of the formula (I), there can be used 2-naphthol, 7-hydroxy-2-naphthol, 2-naphthol-7-sulfonic acid, 2-naphthol-3,6-disulfonic acid, 2-naphthol-3-carboxylic acid, etc.

As phenol and derivatives thereof used as developer, there can be used phenol, p-chlorophenol, 2,4-dichlorophenol, p-bromophenol, o-chlorophenol, m-chlorophenol, and the like.

An aniline and derivatives thereof used as developer, there can be used aniline, N,N-dimethylaniline, N,N-diethylaniline, N,N-diethyl-m-toluidine, 3-methyl-N-ethyl-N-(β-hydroxyethyl)-aniline, N-ethyl-N-(2-hydroxy-3-sulfopropyl)-m-toluidine, 3,5-dimethyl-N-ethyl-N-(2-hydroxy-3-sulfopropyl)-aniline, 3,5-dimethoxy-N-ethyl-(2-hydroxy-3-sulfopropyl)-aniline, and the like.

As 1-naphthol and derivatives thereof used as developer, there can be used 1-naphthol, 1-naphthol-2-sulfonic acid, 1-naphthol-2-carboxylic acid, 1-naphthol-8-sulfonic acid, 1-naphthol-3-sulfonic acid, 1-naphthol-5-sulfonic acid, and the like.

As the coupler, there can be used 4-aminoantipyrine, etc.

The first reagent solution comprises a compound of the formula (I) in an amount of usually 0.01 to 10 mM, preferably 0.1 to 0.5 mM, a peroxidase in an amount of usually 0.1—10 units/ml, an oxidase in an amount of usually 0.05—20 units/ml and, if necessary one or more substances for producing a substrate for the oxidase, used as a substrate for the oxidase, or used for an enzymatic reaction of a substrate with the oxidase depending on the kind of substrate or enzyme to be determined.

The second reagent solution comprises a coupler such as 4-aminoantipyrine, a developer selected from phenol and derivatives thereof, 1-naphthol and derivatives thereof and aniline and derivatives thereof, and an enzyme necessary for producing the same substance as giving an error to the determination in the case of determining the substrate, or one or more substances for giving a substrate for the enzyme, the activity of which is to be determined, in the case of determining the enzymatic activity. The enzyme necessary for producing the same substance as giving an error to the determination of substrate changes depending on the kind of substrate to be determined. The substances for giving a substrate for the enzyme, the activity of which is to be determined, changes depending on the kind of enzyme to be determined its activity.

The first reagent solution and the second reagent solution may further contain, respectively, one or more buffering agents, surface active agents, stabilizers, and the like additives depending on purposes. As the buffering agents, there can be used conventionally used buffer solutions. The pH of the first and second reagent solutions is usually in the range of 5 to 9.

The time necessary for decomposing hydrogen peroxide with the compound of the formula (I) to form a non-coloring compound was measured as follows.

To 3 ml of a Good's buffer solution having a pH 7, 10 units of peroxidase and 0.2 mM of the compound of the formula (I) were added. Subsequently, hydrogen peroxide was added to the resulting mixture so as to make the content of hydrogen peroxide 0.1 mM in the reaction sample, followed by incubation at 37°C for a predetermined time. The time required for decomposing hydrogen peroxide (for discoloring) was measured by adding 0.3 mg of 4-aminoantipyrine and 3 mg of phenol to the mixture after the incubation to measure the production of color. The results are as shown in Table 1. As is clear from Table 1, the time required for discoloring is very short.

Table 1

| Compound of the formula (I) | Time required for discoloring (sec) |
|---|---|
| 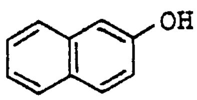 | 5 |
| 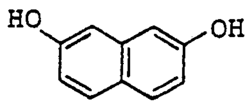 | 5 |
| 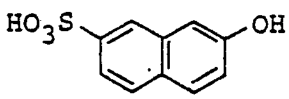 | 5 |
| 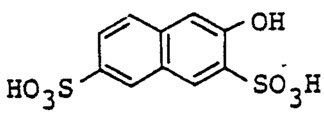 | 5 |
| 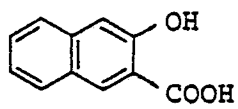 | 5 |

Further, there was admitted almost no influence of the compound of the formula (I) on color producing by the oxidative condensation between phenol or a derivative thereof, or aniline or a derivative thereof or 1-naphthol or a derivative thereof and 4-aminoantipyrine. Such experimental results are shown in Table 2. The experiments were conducted as follows. To 3 ml of 50 mM tris-HCl buffer solution (pH 6.5) containing a combination of developer as shown in Table 2 and peroxidase (10 units), 20 µl of a 10 mM solution of hydrogen peroxide was added and changes of degree of coloring were measured at near the maximum absorption wavelengths of the oxidative condensation products in the absence or presence of the compound of the formula (I) in an amount of 0.5 mM as listed in Table 2. As is clear from Table 2, there was admitted no difference in the absorptions depending on the addition or non-addition of the compound of the formula (I). In Table 2, MEHA means 3-methyl-N-ethyl-N-(β-hydroxyethyl)-aniline and DAOS means 3,5-dimethoxy-N-ethyl-N-(2-hydroxy-3-sulfopropyl)-aniline. 4-Aminoantipyrine was used in an amount of 0.4 mM and phenol and 1-naphthol-2-sulfonic acid were used in an amount of 5 mM, respectively, and MEHA and DAOS were used in an amount of 1 mM, respectively.

Table 2

| Coupler / Developer / Measuring wavelength (nm) / Compound of the formula (I) | 4-Aminoantipyrine | | | |
|---|---|---|---|---|
| Developer | Phenol | 1-Naphthol-2-sulfonic acid | MEHA | DAOS |
| Measuring wavelength (nm) | 505 | 500 | 550 | 600 |
| No addition | 0.425 | 0.420 | 1.05 | 0.511 |
| 2-Naphthol | 0.425 | 0.420 | 1.04 | 0.508 |
| 2-Naphthol-7-sulfonic acid | 0.424 | 0.419 | 1.05 | 0.507 |
| 2-Naphthol-3-carboxylic acid | 0.424 | 0.420 | 1.05 | 0.510 |

In the above, all the concentration of reagents mean final concentrations. (The same thing can be applied to the values mentioned below.)

Detailed explanations will be given below on quantitative determinations of substrates or enzymatic activities.

(1) Triglyceride (interfering substance: glycerol)

$$\text{triglyceride} + 3H_2O \xrightarrow{\text{LPL}} \text{glycerol} + 3\ \text{fatty acid}$$

$$\text{glycerol} + \text{ATP} \xrightarrow[\text{Mg}^{++}]{\text{GK}} \text{glycerol-3-phosphate} + \text{ADP}$$

$$\text{glycerol-3-phosphate} + O_2 \xrightarrow{\text{GPO}} \text{dihydroxyacetone phosphate} + H_2O_2$$

$$H_2O_2 + \text{2-naphthol (I)} \xrightarrow{\text{POD}} \text{non-coloring substance}$$

$$H_2O_2 + \text{4-aminoantipyrine} + \text{developer} \xrightarrow{\text{POD*}} \text{color development}$$

LPL: lipoprotein lipase
GK: glycerol kinase
ATP: adenosine-5'-triphosphate
ADP: adenosine diphosphate
GPO: glycerol-3-phosphate oxidase
POD: peroxidase
POD*: residual peroxidase

(A) First reagent solution

| | |
|---|---|
| buffer solution | pH 6 to 7 |
| 2-naphthol compound (I) | 0.01 to 10 mM |
| peroxidase (POD) | 0.1 to 10 units/ml |
| oxidase (i.e. GPO) | 1 to 20 units/ml |
| substance for producing a substrate for the oxidase | ATP 0.5 to 10 mM<br>GK 0.5 to 10 units/ml<br>Mg acetate 0.3 to 3 mM |

(B) Second reagent solution

| | |
|---|---|
| buffer solution | pH 6 to 7 |
| 4-aminoantipyrine | 0.1 to 3 mM |
| developer | 0.1 to 20 mM |
| enzyme necessary for producing the same substance as the interfering substance | { LPL<br>20 to 200 units/ml |

A sample such as serum is added to the first reagent solution and incubated, for example, at 37°C for 5 minutes. Free glycerol present in the serum is reacted with ATP in the presence of GK and $Mg^{++}$, followed by reaction with $O_2$ in the presence of GPO to give $H_2O_2$, which is reacted with the 2-naphthol compound in the presence of peroxide (POD) to give non-coloring substance. Then, the second reagent solution is added to the resulting mixture and incubated, for example, at 37°C for 10 minutes to hydrolyze triglyceride in the presence of LPL to give glycerol, which is reacted to finally yield $H_2O_2$, the amount of which is measured colorimetrically at a wavelength of e.g. 600 nm, by a conventional method.

(2) Cholesterol ester (interfering substance: cholesterol)

$$\text{cholesterol ester} + H_2O \xrightleftharpoons{\text{cholesterol esterase}} \text{cholesterol} + \text{fatty acid}$$

$$\text{cholesterol} + O_2 \xrightarrow{\text{cholesterol oxidase}} \Delta^4\text{-cholestenone} + H_2O_2$$

$$H_2O_2 + \text{2-naphthol (I)} \xrightarrow{\text{POD}} \text{non-coloring substance}$$

$$H_2O_2 + \text{4-aminoantipyrine} + \text{developer} \xrightarrow{\text{POD*}} \text{color development}$$

(A) First reagent solution

| | |
|---|---|
| buffer solution | pH 6.5 to 7.5 |
| 2-naphthol compound (I) | 0.01 to 10 mM |
| peroxidase (POD) | 0.1 to 10 units/ml |
| oxidase<br>(i.e. cholesterol oxidase) | 0.1 to 10 units/ml |

(B) Second reagent solution

| | |
|---|---|
| buffer solution | pH 6.5—7.5 |
| 4-aminoantipyrine | 0.1 to 3 mM |
| developer | 0.1 to 20 mM |
| enzyme necessary for producing the same substance as the interfering substance | { cholesterol esterase<br>0.1 to 10 units/ml |

A sample is added to the first reagent solution and incubated to decompose the interfering substance, i.e., cholesterol, in the same manner as mentioned above, followed by addition of the second reagent solution to the resulting mixture and incubation for the colorimetrical determination in the same manner as mentioned above.

(3) Creatine (interfering substance: sarcosine)

$$\text{creatine} + H_2O \xrightarrow{\text{creatine amidinohydrase}} \text{sarcosine} + \text{urea}$$

$$\text{sarcosine} + H_2O + O_2 \xrightarrow{\text{sarcosine oxidase}} H_2O_2 + HCHO + \text{glycine}$$

$$H_2O_2 + \text{2-naphthol (I)} \xrightarrow{\text{POD}} \text{non-coloring substance}$$

$$H_2O_2 + \text{4-aminoantipyrine} + \text{developer} \xrightarrow{\text{POD*}} \text{color development}$$

(A) First reagent solution

| | |
|---|---|
| buffer | pH 7.5 to 8.5 |
| 2-naphthol compound (I) | 0.01 to 10 mM |
| peroxidase (POD) | 0.1 to 10 units/ml |
| oxidase (i.e. sarcosine oxidase) | 0.1 to 20 units/ml |

(B) Second reagent solution

| | |
|---|---|
| buffer solution | pH 7.5 to 8.5 |
| 4-aminoantipyrine | 0.1 to 3 mM |
| developer | 0.1 to 20 mM |
| enzyme necessary for producing the same substance as the interfering substance | creatine amidinohydrase 0.1 to 100 units/ml |

A sample is added to the first reagent solution and incubated to decompose the interfering substance, i.e., sarcosine, in the same manner as mentioned above, followed by addition of the second reagent solution to the resulting mixture and incubation for the colorimetric determination in the same manner as mentioned above.

(4) Creatinine (interference substance: sarcosine)

$$\text{creatinine} \xrightarrow{\text{creatininase}} \text{creatine} + H_2O$$

The determination can be carried out in the same manner as described in (3) creatine.

(5) Glutamate pyruvate transaminase activity (interfering substance: pyruvic acid)

$$\text{alanine} + \alpha\text{-ketoglutaric acid} \xrightarrow{\text{GPT}} \text{glutaric acid} + \text{pyruvic acid}$$

$$\text{pyruvic acid} + O_2 + TPP + FAD \xrightarrow[Mg^{++}]{\text{pyruvate oxidase}} \text{acetylphosphate} + CO_2 + H_2O_2$$

$$H_2O_2 + \text{2-naphthol (I)} \xrightarrow{\text{POD}} \text{non-coloring substance}$$

$$H_2O_2 + \text{4-aminoantipyrine} + \text{developer} \xrightarrow{POD^*} \text{color development}$$

GPT: glutamate pyruvate transaminase
TPP: thiamine pyrophosphate
FAD: flavin adenine dinucleotide

(A) First reagent solution

| | |
|---|---|
| buffer solution | pH = 7 to 8 |
| 2-naphthol compound (I) | 0.01 to 10 mM |
| peroxidase (POD) | 0.1 to 10 units/ml |
| oxidase (i.e. pyruvate oxidase) | 0.1 to 10 units/ml |
| substances used for an enzymatic reaction of a substrate with the oxidase | TPP 0.001 to 0.5%<br>FAD 0.01 to 1 mM<br>Mg acetate 0.1 to 10 mM |

(B) Second reagent solution

| | |
|---|---|
| buffer solution | pH = 7 to 8 |
| 4-aminoantipyrine | 0.1 to 3 mM |
| developer | 0.1 to 20 mM |
| substances used as substrates for the enzyme to be determined | DL-alanine 200 to 800 mM<br>α-ketoglutaric acid 2 to 20 mM |

A sample is added to the first reagent solution and incubated to decompose the interfering substance, i.e., pyruvic acid, in the same manner as mentioned above, followed by addition of the second reagent solution to the resulting mixture and incubation for the colorimetrical determination in the same manner as mentioned above.

(6) Glutamate oxaloacetate transaminase activity (interfering substance: oxaloacetic acid, pyruvic acid)

$$\text{aspartic acid} + \alpha\text{-ketoglutaric acid} \overset{GOT}{\rightleftharpoons} \text{glutamic acid} + \text{oxaloacetic acid}$$

$$\text{oxaloacetic acid} \xrightarrow{OAC} \text{pyruvic acid} + CO_2$$

$$\text{pyruvic acid} + O_2 + TPP + FAD \xrightarrow[Mg^{++}]{\text{pyruvate oxidase}} \text{acetylphosphate} + CO_2 + H_2O_2$$

$$H_2O_2 + \text{2-naphthol (I)} \xrightarrow{POD} \text{non-coloring substance}$$

$$H_2O_2 + \text{4-aminoantipyrine} + \text{developer} \xrightarrow{POD^*} \text{color development}$$

GOT: glutamate oxaloacetate transaminase
OAC: oxaloacetate decarboxylase
TPP: thiamine pyrophosphate
FAD: flavin adenine dinucleotide

(A) First reagent solution

| | |
|---|---|
| buffer solution | pH 7 to 8 |
| 2-naphthol compound (I) | 0.01 to 10 mM |
| peroxidase (POD) | 0.1 to 10 units/ml |
| oxidase (i.e. pyruvate oxidase) | 1 to 10 units/ml |
| substances for producing a substrate for the oxidase | OAC 1 to 50 units/ml |
| substances used for an enzymatic reaction of a substrate with the oxidase | TPP 0.001 to 0.5%<br>FAD 0.01 to 1 mM<br>Mg acetate 0.1 to 10 mM |

(B) Second reagent solution

| | |
|---|---|
| buffer solution | pH 7 to 8 |
| 4-aminoantipyrine | 0.1 to 3 mM |
| developer | 0.1 to 20 mM |
| substances used as substrates for the enzyme to be determined | aspartic acid 50 to 400 mM<br>α-ketoglutaric acid 2 to 20 mM |

A sample is added to the first reagent solution and incubation to decompose the interfering substance, i.e., oxaloacetic acid and pyruvic acid, in the same manner mentioned above, followed by addition of the second reagent solution to the resulting mixture and incubation for the colorimetric determination in the same manner as mentioned above.

(7) α-Amylase activity (interfering substance: glucose)

$$\text{short chain amylose} \xrightarrow{\alpha\text{-amylase}} \text{maltose}$$

$$\text{maltose} \xrightarrow{\alpha\text{-glucosidase}} \text{glucose}$$

$$\text{glucose} + O_2 + H_2O \xrightarrow{\text{glucose oxidase}} \text{gluconic acid} + H_2O_2$$

$$H_2O_2 + \text{2-naphthol (I)} \xrightarrow{\text{POD}} \text{non-coloring substance}$$

$$H_2O_2 + \text{4-aminoantipyrine} + \text{developer} \xrightarrow{\text{POD*}} \text{color development}$$

(A) First reagent solution

| | |
|---|---|
| buffer solution | pH 6 to 8 |
| 2-naphthol compound (I) | 0.01 to 10 mM |
| peroxidase (POD) | 0.1 to 10 units/ml |
| oxidase (i.e. glucose oxidase) | 1 to 100 units/ml |

(B) Second reagent solution

| | |
|---|---|
| buffer solution | pH 6 to 8 |
| 4-aminoantipyrine | 0.1 to 3 mM |
| developer | 0.1 to 20 mM |
| substances used as substrates for the enzyme to be determined | short chain amylose 1 to 20 mg/ml (glucose units: 4—6) |
| substance for producing a substrate for the oxidase | α-glucosidase 1 to 100 units/ml |

A sample is added to the first reagent solution and incubated to decompose the interfering substances, i.e., glucose, in the same manner as mentioned above, followed by addition of the second reagent solution to the resulting mixture and incubation for the colorimetric determination in the same manner as mentioned above.

As mentioned above, substrates or enzyme activities in body fluid samples can be determined quantitatively with high accuracy according to this invention without any influence of interfering substances present in the samples.

This invention is illustrated by way of the following Examples.

Example 1
[Determination of Trilyceride]

(1) Reagent Solutions

(A) First reagent solution:

| | |
|---|---|
| 0.05 M tris-HCl buffer solution | (pH 6.5) |
| glycerol kinase | 15 units/ml |
| glycerol-3-phosphate oxidase | 15 units/ml |
| peroxidase | 10 units/ml |
| 2-naphthol-7-sulfonic acid | 0.5 mM |
| magnesium acetate | 5 mM |
| adenosine-5′-triphosphate | 6 mM |

(B) Second reagent solution:

| | |
|---|---|
| 0.05 M tris-HCl buffer solution | (pH 6.5) |
| lipoprotein lipase | 100 units/ml |
| 4-aminoantipyrine | 0.2 mM |
| 3,5-dimethoxy-N-ethyl-(2-hydroxy-3-sulfopropyl)-aniline | 1 mM |

(2) Measuring Method

To 20 μl of a sample (serum), 1 ml of the first reagent solution was added with well mixing and incubated at 37°C for 5 minutes. Subsequently, the second reagent solution was added to the resulting mixture and incubated at 37°C for 10 minutes. A reagent blank was prepared in the same manner as mentioned above using 20 μl of distilled water. Absorbance of the serum sample was measured at the wavelength of 600 nm using the reagent blank as control.

On the other hand, absorbances of control serum containing known amounts of triglyceride were measured in the same manner as mentioned above. The true content of triglyceride in the serum sample was obtained by proportional calculation containing no error derived from free glycerol in the sample.

In order to know an influence of free glycerol on the colorimetrical determination, known amounts of glycerol were added to human serum samples, which were treated in the same manner as mentioned

above. The results of the colorimetrical determination are shown in Table 3. As shown in Table 3, no influence of free glycerol on the measurement was admitted.

Table 3

| Run No. | Added amount of glycerol | Measured value of triglyceride |
|---|---|---|
| | (mg/dl) | (mg/dl) |
| 1 | 0 | 186 |
| 2 | 10 | 185 |
| 3 | 20 | 186 |
| 4 | 30 | 186 |
| 5 | 60 | 186 |

Example 2
[Determination of GPT activity]

(1) Reagent Solutions
(A) First reagent solution:

| | |
|---|---|
| 0.02 M phosphate buffer solution | (pH 7.0) |
| pyruvate oxidase | 6 units/ml |
| peroxidase | 10 units/ml |
| thiamine pyrophosphate | 0.06% by weight |
| flavin adenine dinucleotide | 0.002% by weight |
| magnesium acetate | 9 mM |
| 2-naphthol-3-carboxylic acid | 0.5 mM |

(B) Second reagent solution:

| | |
|---|---|
| 0.02 M phosphate buffer solution | (pH 7.0) |
| α-ketoglutaric acid | 35 mM |
| DL-alanine | 700 mM |
| 4-aminoantipyrine | 50 mg/dl |
| 3-methyl-N-ethyl-N-(β-hydroxyethyl)-aniline | 50 mg/dl |

(C) Reaction stopper solution:

| | |
|---|---|
| 0.05 M phosphate buffer solution | (pH 9.0) |
| 2-sodium ethylene diaminetetraacetate | 0.05 M |
| 3-sodium citrate | 0.1 M |

(2) Measuring Method

To 20 µl of a sample (serum), 0.5 ml of the first reagent solution was added with well mixing and incubated at 37°C for 5 minutes. Subsequently, 0.2 ml of the second reagent solution was added to the resulting mixture with well mixing and incubated at just 37°C for 20 minutes, followed by addition of 2 ml of the reaction stopper solution. A reagent blank was prepared in the same manner as mentioned above using 20 µl of distilled water. Absorbance of the serum sample was measured at the wavelength of 560 nm using the reagent blank as control.

On the other hand, absorbances of control serum having known activities of GPT were measured in the same manner as mentioned above. True GPT activity values in the serum sample were obtained by proportional calculation containing no error derived from free pyruvic acid in the sample.

Example 3
[Determination of GOT activity]

(1) Reagent Solutions

(A) First reagent solution:

| | |
|---|---|
| 0.02 M phosphate buffer solution | (pH 7.0) |
| pyruvate oxidase | 6 units/ml |
| peroxidase | 10 units/ml |
| oxaloacetate decarboxylase | 10 units/ml |
| thiamine pyrophosphate | 0.06% by weight |
| flavin adenine dinucleotide | 0.002% by weight |
| magnesium acetate | 9 mM |
| 2-naphthol-3-carboxylic acid | 0.5 mM |

(B) Second reagent solution:

| | |
|---|---|
| 0.02 M phosphate buffer solution | (pH 7.0) |
| α-ketoglutaric acid | 35 mM |
| L-aspartic acid | 280 mM |
| 4-aminoantipyrine | 50 mg/dl |
| 3-methyl-N-ethyl-N-(β-hydroxyethyl)-aniline | 50 mg/dl |

(C) Reaction stopper solution:

| | |
|---|---|
| 0.05 M phosphate buffer solution | (pH 9.0) |
| 2-sodium ethylenediaminetracetate | 0.05 M |
| 3-sodium citrate | 0.1 M |

(2) Measuring method

To 20 µl of a sample (serum), 0.5 ml of the first reagent solution was added with well mixing and incubated at 37°C for 5 minutes. Subsequently, 0.2 ml of the second reagent solution was added to the resulting mixture with well mixing and incubated at just 37°C for 20 minutes, followed by addition of 2 ml of the reaction stopper solution. A reagent blank was prepared in the same manner as mentioned above using 20 µl of distilled water. Absorbance of the serum sample was measured at the wavelength of 560 nm using the reagent blank as control.

On the other hand, absorbances of control serum having known activities of GOT were measured in the same manner as mentioned above. True GOT activity values in the serum sample were obtained by proportional calculation containing no error derived from pyruvic acid in the sample.

Example 4
[Determination of cholesterol ester]

(1) Reagent Solutions

(A) First reagent solution:

| | |
|---|---|
| 0.10 M phosphate buffer solution | (pH 7.0) |
| cholesterol oxidase | 0.3 units/ml |
| peroxidase | 10 units/ml |
| Triton X—100 | 0.10% by weight |
| 2-naphthol-7-sulfonic acid | 0.33 mM |

(B) Second reagent solution:

| | |
|---|---|
| 0.10 M phosphate buffer solution | (pH 7.0) |
| cholesterol esterase | 0.3 unit/ml |
| Triton X—100 | 0.10% by weight |
| 4-aminoantipyrine | 30 mg/dl |
| phenol | 200 mg/dl |

[Triton X—100 = octyl phenoxy polyethoxyethanol — available from Rohm and Haas Co.]

(2) Measuring Method

To 20 μl of a sample (serum), 1.5 ml of the first reagent solution was added with well mixing and incubated at 37°C for 15 minutes. Subsequently, the second reagent solution was added to the resulting mixture with well mixing and incubated at 37°C for 15 minutes. A reagent blank was prepared in the same manner as mentioned above using 20 μl of distilled water. Absorbance of the serum sample was measured at the wavelength of 505 nm using the reagent blank as control.

On the other hand, absorbances of control serum containing known amounts of cholesterol ester were measured in the same manner as mentioned above. The true content of cholesterol ester in the serum sample was obtained by proportional calculation containing no error derived from free cholesterol in the sample.

**Claims**

1. A process for quantitatively determining a substrate or enzymatic activity in a body fluid sample which comprises adding to a sample to be measured

— a first reagent solution comprising

(i) a compound of the formula (I):

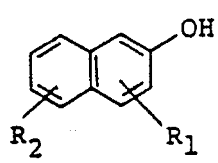

(I)

wherein $R_1$ and $R_2$ are independently hydrogen, a hydroxyl group, a carboxyl group or a sulfonic acid group,

(ii) a peroxidase,

(iii) an oxidase, and

(iv) if necessary one or more substances used for producing a substrate or used as substrates for the oxidase or used for an enzymatic reaction of a substrate with the oxidase so as to remove an influence of substance in the sample giving an error to the determination,

adding to the resulting mixture

— a second reagent solution comprising

(v) a coupler,

(vi) a developer selected from phenol and derivatives thereof, 1-naphthol and derivatives thereof and aniline and derivatives thereof, and

(vii) an enzyme necessary for producing the same substance as giving an error to the determination in the case of determining the substrate, or one or more substances used as substrates for the enzyme, the activity of which is to be determined, in the case of determining the enzymatic activity,
and then performing a colorimetric determination.

2. A process according to Claim 1, wherein the compound of the formula (I) is 2-naphthol, 7-hydroxy-2-naphthol, 2-naphthol-7-sulfonic acid, 2-naphthol-3,6-disulfonic acid or 2-naphthol-3-carboxylic acid.

3. A process according to Claim 1, wherein the substrate to be determined is triglyceride, the oxidase in the first reagent solution is glycerol-3-phosphate oxidase, the substances for producing a substrate for the oxidase in the first reagent solution are adenosine-5'-triphosphate, glycerol kinase and magnesium acetate, and the enzyme necessary for producing the same substance as giving an error to the determination in the second reagent solution is lipoprotein lipase.

4. A process according to Claim 1, wherein the substrate to be determined is cholesterol ester, the oxidase in the first reagent solution is cholesterol oxidase, and the enzyme necessary for producing the same substance as giving an error to the determination in the second reagent solution is cholesterol esterase.

5. A process according to Claim 1, wherein the substrate to be determined is creatine, the oxidase in the first reagent solution is sarcosine oxidase, and the enzyme necessary for producing the same substance as giving an error to the determination in the second reagent solution is creatine amidinohydrase.

6. A process according to Claim 1, wherein the substrate to be determined is creatinine.

7. A process according to Claim 1, wherein the enzyme the activity of which is to be determined is glutamate pyruvate transaminase, the oxidase in the first reagent solution is pyruvate oxidase, the substances used for an enzymatic reaction of a substrate with the oxidase in the first reagent solution are thiamine pyrophosphate and flavin adenine dinucleotide, and the substances used as substrates for the enzyme to be determined are DL-alanine and α-ketoglutaric acid.

8. A process according to Claim 1, wherein the enzyme the activity of which is to be determined is glutamate oxaloacetate transaminase, the oxidase in the first reagent solution is pyruvate oxidase, the substance used for producing a substrate fo the oxidase in the first reagent solution is oxaloacetate decarboxylase, the substances used for an enzymatic reaction of a substrate for the oxidase are thiamine pyrophosphate and flavin adenine dinucleotide, and the substances used as substrates for the enzyme to be determined are aspartic acid and α-ketoglutaric acid.

9. A process according to Claim 1, wherein the enzyme the activity of which is to be determined is α-amylase, the oxidase in the first reagent solution is glucose oxidase, and the substance used as substrate for the enzyme to be determined is short chain amylose and the substance used for producing a substrate for the oxidase is α-glucosidase.

**Patentansprüche**

1. Verfahren zur quantitativen Bestimmung eines Substrates oder einer Enzymaktivität in einer Körperflüssigkeitsprobe, wobei man zu einer zu messenden Probe
— eine erste Reagenzlösung gibt, umfassend
(i) eine Verbindung der Formel (I):

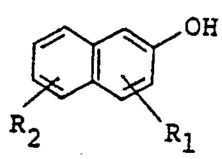

(I)

worin $R_1$ und $R_2$ unabhängig voneinander Wasserstoff, eine Hydroxylgruppe, eine Carboxylgruppe oder eine Sulfonsäuregruppe bezeichnen,
(ii) eine Peroxidase,
(iii) eine Oxidase, und
(iv) wenn nötig, eine oder mehrere Substanzen, die verwendet werden zur Herstellung eines Substrates, oder die benützt werden als Substrate für die Oxidase, oder die gebraucht werden für eine enzymatische Reaktion eines Substrates mit der Oxidase, so daß ein Einfluß der Substanz in der probe, der einen Fehler in der Bestimmung bewirkt, ausgeschaltet wird,
und wobei man zu der entstandenen Mischung
— eine zweite Reagenslösung gibt, umfassend
(v) einen Kuppler,
(vi) einen Entwickler, ausgewählt unter Phenol und Derivaten davon, 1-Naphthol und Derivaten davon und Anilin und Derivaten davon, und
(vii) ein Enzym, welches im Falle der Substratbestimmung notwendig ist zur Hestellung der gleichen Substanz, welche einen Fehler bei der Bestimmung verursacht, oder ein oder mehrere Substanzen, welche im falle der Bestimmung der Enzymaktivität als Substrate für das Enzym verwendet werden, dessen Aktivität zu bestimmen ist,
und dann eine kolorimetrische Bestimmung durchführt.

2. Verfahren nach Anspruch 1, wobei die Verbindung der Formel (I) 2-Naphthol, 7-Hydroxy-2-naphthol, 2-Naphthol-7-sulfonsäure, 2-Naphthol-3,6-disulfonsäure oder 2-Naphthol-3-carbonsäure ist.

3. Verfahren nach Anspruch 1, wobei das zu bestimmende Substrat ein Triglycerid ist, die Oxidase in der ersten Reagenzlösung Glycerin-3-phosphatoxidase ist, die Substanzen zur Herstellung eines Substrates für die Oxidase in der ersten Reagenzlösung Adenosin-5-triphosphat, Glycerinkinase und Magnesiumacetat sind, und das Enzym in der zweiten Reagenzlösung, welches notwendig ist für die Herstellung der gleichen Substanz, die bei der Bestimmung einen Fehler verursacht, Lipoproteinlipase ist.

4. Verfahren nach Anspruch 1, wobei das zu bestimmende Substrat ein Cholesterinester ist, die Oxidase in der ersten Reagenzlösung Cholesterinoxidase ist, und das Enzym in der zweiten Reagenzlösung, welches notwendig ist für die Herstellung der gleichen Substanz, die bei der Bestimmung einen Fehler verursacht, Cholesterinesterase ist.

5. Verfahren nach Anspruch 1, wobei das zu bestimmende Substrat Kreatin ist, die Oxidase in der ersten Reagenzlösung Sarkosinoxidase ist, und das Enzym in der zweiten Reagenzlösung, welches notwendig ist zur Herstellung der gleichen Substanz, die bei der Bestimmung einen Fehler verursacht, Kreatinamidinohydrase ist.

6. Verfahren nach Anspruch 1, wobei das zu bestimmende Substrat Kreatinin ist.

7. Verfahren nach Anspruch 1, wobei das Enzym, dessen Aktivität zu bestimmen ist, Glutamatpyruvattransaminase ist, die Oxidase in der ersten Reagenzlösung Pyruvatoxidase ist, die Substanzen in der ersten Reagenzlösung, welche für eine enzymatische Reaktion eines Substrates mit der Oxidase gebraucht werden, Thiaminpyrophosphat und Flavinadenindinucleotid sind, und die Substanzen, welche als Substrate für das zu bestimmende Enzym gebraucht werden, DL-Alanin und α-Ketoglutarsäure sind.

8. Verfahren nach Anspruch 1, wobei das Enzym, dessen Aktivität bestimmt werden soll, Glutamatoxalacetattransaminase ist, die Oxidase in der ersten Reagenzlösung Pyruvatoxidase ist, die Substanz in der ersten Reagenzlösung, welche für die Herstellung eines Substrates für die Oxidase gebraucht wird, Oxalacetatdecarboxylase ist, die Substanzen, welche für eine enzymatische Reaktion eines Substrates für die Oxidase gebraucht werden, Thiaminpyrophosphat und Flavinadenindinucleotid sind, und die Substanzen, die als Substrate für das zu bestimmende Enzym gebraucht werden, Asparaginsäure und α-Ketoglutarsäure sind.

9. Verfahren nach Anspruch 1, wobei das Enzym, dessen Aktivität bestimmt werden soll, α-Amylase ist, die Oxidase in der ersten Reagenzlösung Glucoseoxidase ist, und die Substanz, welche als Substrat für das zu bestimmende Enzym gebraucht wird, eine kurzkettige Amylose ist, und die Substanz, welche zur Herstellung eines Substrates für die Oxidase gebraucht wird, α-Glucosidase ist.

**Revendications**

1. Procédé de détermination quantitative d'un substrat ou d'une activité enzymatique dans un échantillon de fluide corporel qui comprend l'addition à un échantillon à mesurer

— d'une première solution de réactif comprenant

(i) un composé répondant à la formule (I):

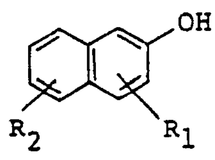

(I)

dans laquelle $R_1$ et $R_2$ sont indépendamment un atome d'hydrogène, un groupe hydroxyle, un groupe carboxyle ou un groupe acide sulfonique,

(ii) une péroxydase,

(iii) une oxydase, et

(iv) si nécessaire une ou plusieurs substances utilisées pour produire un substrat ou utilisées comme substrats pour l'oxydase ou utilisées pour une réaction enzymatique d'un substrat avec l'oxydase de manière à éliminer l'influence d'une substance dans l'échantillon conduisant à une détermination erronée, l'addition au mélange obtenu,

— d'une seconde solution de réactif comprenant

(v) un coupleur,

(vi) un révélateur choisi parmi le phénol et des dérivés de celui-ci, et l'aniline et des dérivés de celle-ci, le 1-naphtol et des dérivés de celui-ci, et

(vii) une enzyme nécessaire pour produire cette substance conduisant à une détermination erronée dans le cas d'une détermination du substrat, ou une ou plusieurs substances utilisées comme substrats pour l'enzyme, dont l'activité doit être déterminée, dans le cas de la détermination de l'activité enzymatique, puis le fait d'effectuer une détermination colorimétrique.

2. Procédé suivant la revendication 1, dans lequel le composé de la formule (I) est le 2-naphtol, le 7-hydroxy-2-naphtol, l'acide 2-naphtol-7-sulfonique, l'acide 2-naphtol-3,6-disulfonique ou l'acide 2-naphtol-3-carboxylique.

3. Procédé suivant la revendication 1, dans lequel le substrat à déterminer est un triglycéride, l'oxydase présente dans la première solution de réactif est la glycérol-3-phosphate oxydase, les substances pour produire un substrat pour l'oxydase dans la première solution de réactif sont l'adénosine-5'-triphosphate, la glycérol kinase et l'acétate de magnésium, et l'enzyme nécessaire pour produire la substance conduisant à une erreur dans la détermination présente dans la seconde solution de réactif est la lipoprotéine lipase.

4. Procédé suivant la revendication 1, dans lequel le substrat à déterminer est un ester de cholestérol, l'oxydase présente dans la première solution de réactif est la cholestérol oxydase, et l'enzyme nécessaire pour produire la substance conduisant à une erreur de détermination présente dans la seconde solution de réactif est la cholestérol estérase.

5. Procédé suivant la revendication 1, dans lequel le substrat à déterminer est la créatine, l'oxydase présente dans la première solution de réactif est la sarcosine oxydase et l'enzyme nécessaire pour produire la même substance conduisant à une erreur de détermination présente dans la seconde solution de réactif est la créatine amidinohydrase.

6. Procédé suivant la revendication 1, dans lequel le substrat à déterminer est la créatinine.

7. Procédé suivant la revendication 1, dans lequel l'enzyme dont l'activité doit être déterminée est la glutamate pyruvate transaminase, l'oxydase présente dans la première solution de réactif est la pyruvate oxydase, les substances utilisées pour la réaction enzymatique d'un substrat avec l'oxydase dans la première solution de réactif sont le pyrophosphate de thiamine et le flavine adénine dinucléotide, et les substances utilisées comme substrats pour l'enzyme à déterminer sont la DL-alanine et l'acide α-cétoglutarique.

8. Procédé suivant la revendication 1, dans lequel l'enzyme dont l'activité doit être déterminée est la glutamate oxaloacétate transaminase, l'oxydase présente dans la première solution de réactif est la pyruvate oxydase, la substance utilisée pour produire un substrat pour l'oxydase dans la première solution de réactif est l'oxaloacétate décarboxylase, les substances utilisées pour la réaction enzymatique d'un substrat pour l'oxydase sont le pyrophosphate de thiamine et le flavine adénine dinucléotide, et les substances utilisées comme substrats pour l'enzyme à déterminer sont l'acide aspartique et l'acide α-cétoglutarique.

9. Procédé suivant la revendication 1, dans lequel l'enzyme dont l'activité doit être déterminée est l'α-amylase, l'oxydase présente dans la première solution de réactif est la glucose oxydase et la substance utilisée comme substrat pour l'enzyme à déterminer est un amylose à chaîne courte et la substance utilisée pour produire un substrat pour l'oxydase est l'α-glucosidase.